# EUROPEAN PATENT SPECIFICATION

(11) **EP 4 140 523 B1**
(45) Date of publication and mention of the grant of the patent: **20.08.2025**
(21) Application number: 22191692.7
(22) Date of filing: 23.08.2022
(51) Int. Cl.: A61M 13/00, B03C 3/017, B03C 3/019, B03C 3/32, B03C 3/41, A61B 18/00

(54) **CLOSED LOOP SMOKE FILTRATION SYSTEM FOR USE IN A LAPAROSCOPIC SURGICAL PROCEDURE**
RAUCHFILTRATIONSSYSTEM MIT GESCHLOSSENEM REGELKREIS ZUR VERWENDUNG IN EINEM LAPAROSKOPISCHEN CHIRURGISCHEN VERFAHREN
SYSTÈME DE FILTRATION DE FUMÉE EN BOUCLE FERMÉE DESTINÉ À ÊTRE UTILISÉ DANS UNE PROCÉDURE CHIRURGICALE LAPAROSCOPIQUE

(30) Priority: 26.08.2021 US 202163237357 P; 13.07.2022 US 202217863512
(43) Date of publication of application: 01.03.2023
(73) Proprietor: Covidien LP, Mansfield, MA 02048 (US)
(72) Inventor: HOLSTEN, Henry E., North Haven, CT 06473 (US)
(74) Representative: Maschio & Soames IP Ltd

(56) References cited:
- WO-A1-2017/184876
- WO-A1-2018/081587
- WO-A1-2020/087007
- WO-A1-2022/018460
- DE-A1- 102017 006 185
- US-A1- 2010 094 200
- US-A1- 2016 287 817

## Description

### CROSS-REFERENCE TO RELATED APPLICATION

This application claims priority to, and the benefit of, U.S. Provisional Patent Application Serial No. 63/237,357, filed on August 26, 2021.

### FIELD

The disclosure generally relates to systems for performing minimally invasive surgery, and more particularly, to a closed loop smoke filtration system.

### BACKGROUND

Minimally invasive surgery eliminates the need to make a large incision in a patient, thereby reducing discomfort, recovery time, and many of the deleterious side effects associated with traditional open surgery. In laparoscopic procedures, the abdominal cavity is insufflated with an insufflation gas such as, e.g., CO₂, to create a pneumoperitoneum thereby providing access to the underlying organs. A laparoscopic instrument is introduced through a cannula or a trocar accessing the abdominal cavity to perform one or more surgical tasks. The cannula may incorporate a seal to establish a substantially fluid tight seal about the instrument to preserve the integrity of the pneumoperitoneum.

Instruments utilized during a laparoscopic procedure may include lasers, electrocautery, or sonic cutting instruments, which produce smoke and/or an aerosol as a byproduct of treating tissue. Smoke plumes may obscure the clinician's field of vision and the odor generated is unpleasant. Further, the smoke plume may contain infectious agents which may contaminate the operating arena thereby presenting a danger to operating personnel. The chemical vapor may be irritating to the respiratory tract and also may be carcinogenic. The smoke, noxious fumes, and other gases and vapors may include particulates, bacteria, viral elements, and undesirable odors.

The document WO 2020/087007 A1 relates to a smoke evacuation system for use during surgical medical procedures. The system comprises: a surgical apparatus having a fluid conduit therethrough; a vacuum tube fluidly coupled with the fluid conduit; an electrostatic precipitator fluidly coupled with the fluid conduit, the electrostatic precipitator comprising at least one collection surface operable to attract ionized particulate; a vacuum source fluidly coupled with the vacuum tube, wherein the vacuum source is operable to create a flow of fluid through the fluid conduit, the vacuum tube and the electrostatic precipitator, wherein the electrostatic precipitator comprises a collection cell that is electrically charged to at least partially capture oppositely charged particulates in the flow of fluid; and a power source electrically coupled with the electrostatic precipitator.

The document WO 2017/184876 A1 relates to a smoke evacuation system for use during surgical medical procedures. The system comprises: a trocar comprising a tubular hollow body circumscribing a cavity extending through a longitudinal axis of the trocar with a gasket at a first end and an exit port at a second end, the tubular hollow body comprising a first wall circumscribing the cavity and a concentrically spaced apart second wall; a first plate operable to maintain an electric charged disposed within the cavity on the first wall; a second plate operable to maintain an electric charge disposed between the first wall and the spaced apart second wall; an insufflator operable to provide gas to the cavity; and a charging controller operable to provide an electric charge to the first plate and the second plate. The electric charge on the first plate is opposite the electric charge on the second plate. The first plate is operable to negatively charge particles, and the second plate is operable to collect and attract negatively charged air particles.

The document WO 2022/018460 A1 is state of the art according to Article 54(3) EPC. This document relates to an insufflation apparatus for use in surgery comprising: an inlet port for receiving insufflating gas from a source of insufflating gas into the apparatus; an outlet port for delivering insufflating gas from the apparatus to a intracorporeal cavity of a patient; a return port for receiving insufflating gas from the patient; a conditioning unit for conditioning the insufflating gas, the conditioning unit comprising an electrostatic precipitator for removing particulate matter present in the extracted insufflation gas using electrostatic precipitation; a regulating assembly for regulating the flow of gas within the apparatus; and, an extractor unit for extracting the insufflating gas from the cavity of the patient to the return port. The electrostatic precipitator for removing particulate material, such as smoke particles, entrained within the insufflation gas, uses electrostatic precipitation. This may be achieved by ionising the insufflation gas and principally the entrained particulates, using an ionisation electrode, and subsequently passing the electrostatically charged gas through an electrically grounded passage so that the charged particulates become attracted to the passage wall, and thus separate from the insufflation gas.

It would be desirable to provide a safe and efficient smoke evacuation system.

### SUMMARY OF THE INVENTION

The present invention provides a closed loop filtration system for use in a laparoscopic surgical procedure as defined in claim 1. Further preferred embodiment of the invention are defined in the dependent claims.

### SUMMARY OF THE DISCLOSURE

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

In accordance with the disclosure, a closed loop filtration system includes first and second trocars providing sealed access to a body cavity, a power supply, first and second ionizer units electrically coupled to the power supply, and a filter cartridge. The filter cartridge includes an outlet in communication with the first trocar, an inlet in communication with the second trocar, a first electrode disposed downstream of the inlet of the filter cartridge, and a second electrode disposed downstream of the first electrode. The first electrode is electrically coupled to the first ionizer unit to ionize airborne particulate matter flowing therethrough. The second electrode is electrically coupled to the second ionizer unit and configured to attract the airborne particulate matter that is ionized by the first electrode.

In an aspect, the closed loop filtration system may further include a pump connected to the outlet of the filter cartridge to direct a fluid from the second trocar to the filter cartridge.

In another aspect, the closed loop filtration system may further include an insufflation source connector that is coupled to an insufflation source and the first trocar.

In yet another aspect, the insufflation source connector may also be connected to an outlet of the pump.

In still yet another aspect, the first or second electrode may be a mesh.

In still yet another aspect, the filter cartridge may further include a mechanical filter.

In an aspect, the mechanical filter may be a HEPA or ULPA filter.

In another aspect, the power supply may provide an output voltage between about 10k VDC to 30k VDC.

In still yet another aspect, the power supply may be a lithium-ion battery.

In an aspect, the first electrode may be negatively charged.

In accordance with another aspect of the disclosure, a closed loop filtration system includes a first trocar configured to supply a fluid into a body cavity, a second trocar configured to discharge the fluid from the body cavity, a power supply, first and second ionizer units electrically coupled to the power supply, and a filter cartridge. The filter cartridge includes an outlet in communication with the first trocar, an inlet in communication with the second trocar, a first electrode disposed downstream of the inlet of the filter cartridge, and a second electrode disposed downstream of the first electrode. The first electrode is electrically coupled to the first ionizer unit to electrically charge airborne particulate matter passing through the first electrode. The second electrode is electrically coupled to the second ionizer unit and configured to attract the airborne particulate matter that is electrically charged by the first electrode.

In an aspect, the fluid may be an insufflation gas.

In another aspect, the filter cartridge may further include a mechanical filter that is disposed downstream of the second electrode.

In yet another aspect, the closed loop filtration system may further include a pump electrically coupled to the power supply and having a pump inlet in communication with the outlet of the filter cartridge and a pump outlet in communication with the first trocar.

In still yet another aspect, the pump may have an output of about 15 liters per minute.

In still yet another aspect, the first or second electrodes may be formed of stainless steel.

In still yet another aspect, the first or second electrodes may be a mesh.

In an aspect, the closed loop filtration system may further include an insufflation source connector disposed downstream of the pump outlet. The insufflation source connector may be coupled to an insufflation gas source.

In another aspect, the pump may be a diaphragm pump.

In yet another aspect, the power supply may be a lithium-ion battery.

### DESCRIPTION OF THE DRAWINGS

The above and other aspects and features of this disclosure will become more apparent in view of the following detailed description when taken in conjunction with the accompanying drawings wherein like reference numerals identify similar or identical elements.
FIG. 1 is a perspective view of the closed loop smoke filtration system in accordance with the disclosure, illustrating use thereof;
FIG. 2 is a perspective view of a filter cartridge of the closed loop smoke filtration system of FIG. 1;
FIG. 3 is a top view of a mesh grid of the filter cartridge of FIG. 2;
FIG. 4 is a perspective view of a power supply, a pump, and an ionizer unit of a power module of the closed loop smoke filtration system; and
FIG. 5 is a perspective view of a filter cartridge for use with the closed loop smoke filtration system of FIG. 1 in accordance with another aspect of the disclosure.

### DETAILED DESCRIPTION

The closed loop smoke filtration system disclosed herein is described in detail with reference to the drawings, in which like reference numerals designate identical or corresponding elements in each of the several views.

As used herein, the term "distal" refers to the portion that is being described which is farther from a user, while the term "proximal" refers to the portion that is being described which is closer to a user. In addition, the terms parallel and perpendicular are understood to include relative configurations that are substantially parallel and substantially perpendicular up to about + or - 10 degrees from true parallel and true perpendicular. Further, to the extent consistent, any or all of the aspects detailed herein may be used in conjunction with any or all of the other aspects detailed herein.

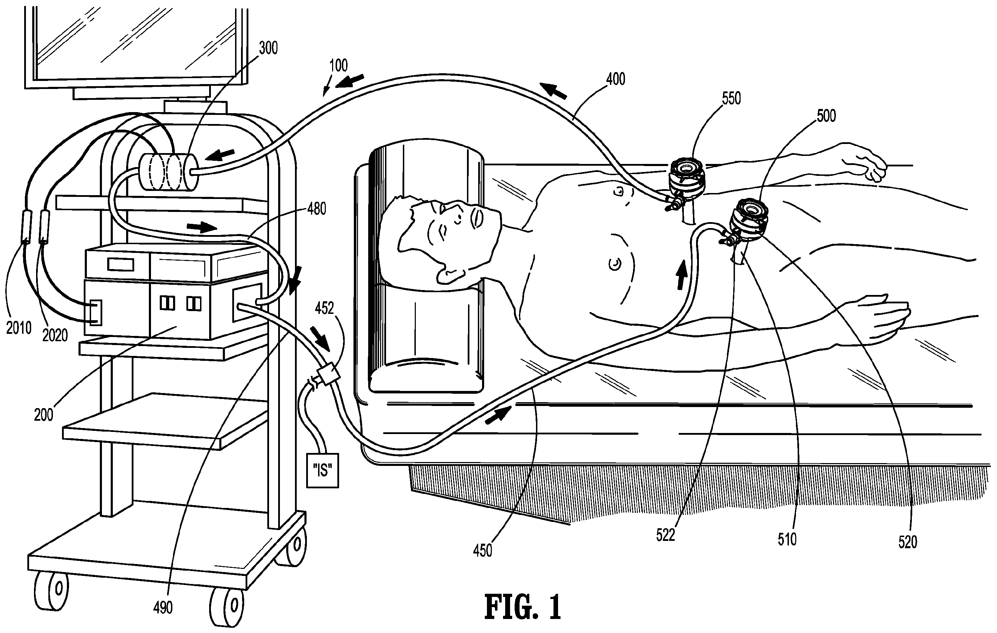

Initially, with reference to FIG. 1, a closed loop smoke filtration system in accordance with the disclosure is shown generally as a smoke filtration system 100. The smoke filtration system 100 inhibits contaminated insufflation gas, which may include airborne particulate matter and pathogens, from being released to the operating room. In particular, the smoke filtration system 100 utilizes an electrostatic precipitator to filter out the airborne particulate matter and pathogens from the contaminated insufflation gas, as will be described. The smoke filtration system 100 includes a power module 200, a filter cartridge 300, an inlet tubing 400, an outlet tubing 450, a connecting inlet tubing 480, a connecting outlet tubing 490, and first and second trocars 500, 550.

The first and second trocars 500, 550 are inserted through tissue and are placed adjacent a surgical site. The first trocar 500 provides sealed access of surgical instruments into an insufflated body cavity, such as the abdominal cavity. The first trocar 500 includes a cannula 510 and a housing 520. The cannula 510 defines a lumen extending therethrough. The housing 520 may be secured with or integrally formed with the cannula 510. The housing 520 is configured to support a seal assembly to provide sealed passage of the surgical instrument through the first trocar 500. The housing 520 includes an insufflation port 522 that is coupled to an insufflation source "IS" to provide the insufflation gas to the body cavity. The outlet tubing 450 interconnects the insufflation port 522 of the first trocar 500 to an insufflation source connector 452 that is coupled to the insufflation source "IS". The insufflation gas from the insufflation source "IS" and the filtered insufflation gas from the filter cartridge 300 are supplied to the body cavity through the first trocar 500. In particular, the insufflation source "IS" provides the insufflation gas to the insufflation source connector 452 to initially insufflate the body cavity and also to compensate for any loss of the insufflation gas during a surgical procedure. While pneumoperitoneum in the body cavity is maintained, the smoke filtration system 100 forms a closed loop system and only the filtered insufflation gas is recirculated through the insufflation source connector 452.

The second trocar 550 is identical to the first trocar 500 and thus will not be described. The second trocar 550 is also placed to provide sealed access of surgical instruments into the insufflated body cavity, such as the abdominal cavity, and to remove the contaminated insufflation gas from the insufflated body cavity. Under such a configuration, new or filtered insufflation gas is supplied to the body cavity via the first trocar 500, while the contaminated insufflation gas is discharged from the body cavity via the second trocar 550 in order to maintain pneumoperitoneum. While the first and second trocars 500, 550 are shown, it is contemplated that a single trocar having separate intake and exhaust flow paths may be used. For a detailed description of the structure and function of components of exemplary surgical access devices such as trocars and cannulas, reference may be made to U.S. Patent Nos. 7,300,448; 7,691,089; and 8,926,508.

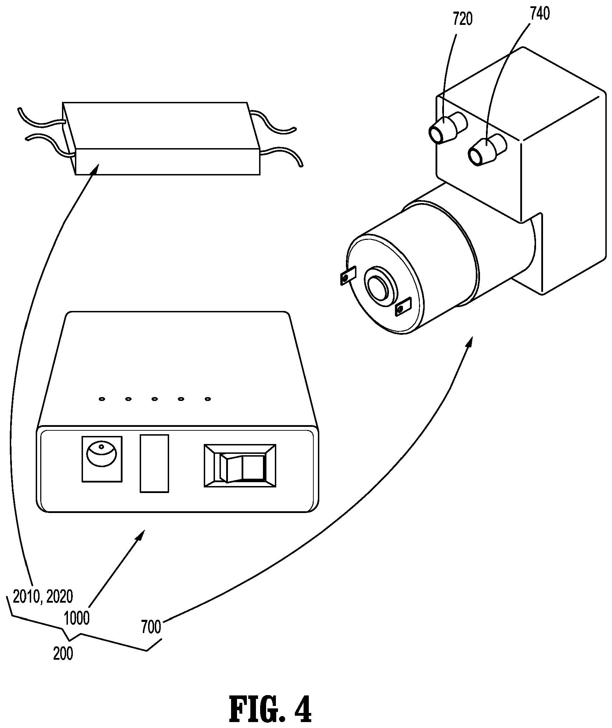

With continued reference to FIG. 1, the contaminated insufflation gas is discharged or evacuated from the second trocar 550 and flows into the filter cartridge 300 via the inlet tubing 400. In an aspect, the filter cartridge 300 may be disposable. The filter cartridge 300 has the electrostatic precipitator that removes particulate matter and pathogens from the contaminated insufflation gas. The filtered insufflation gas then flows into the power module 200 via a connecting inlet tubing 480. The connecting inlet tubing 480 is connected to an inlet port 720 (FIG. 4) of a pump 700 in the power module 200 and a connecting outlet tubing 490 is connected to an outlet port 740 of the pump 700 such that the filtered insufflation gas flows through the pump 700. The connecting outlet tubing 490 interconnects the pump 700 and the insufflation source connector 452 to direct the filtered insufflation gas to the outlet tubing 450. The insufflation source connector 452 merges an insufflation gas from the insufflation source "IS" and the filtered insufflation gas.

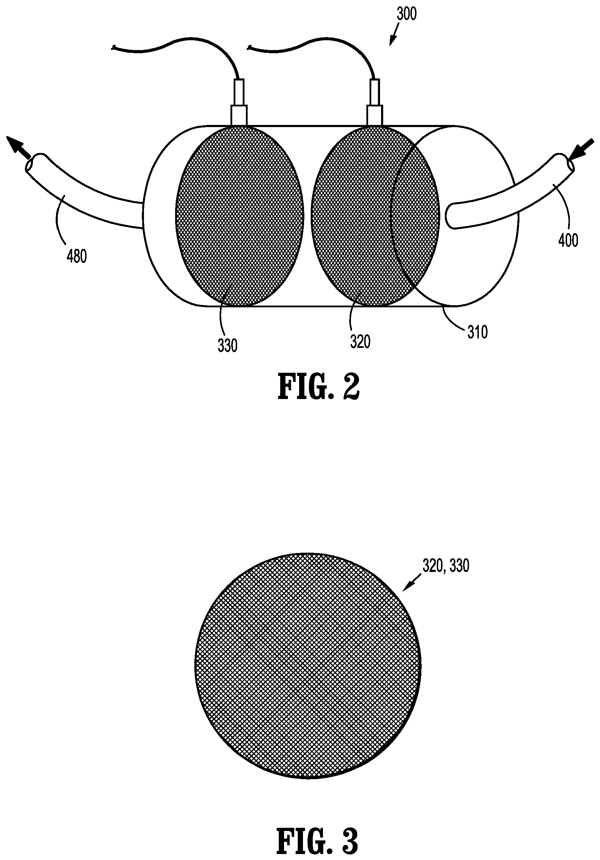

FIG. 2 illustrates the filter cartridge 300 that utilizes the electrostatic precipitator to filter out the particulate matter and pathogens from the contaminated insufflation gas. In particular, the pump 700 (FIG. 4) causes the contaminated insufflation gas to flow from the body cavity to the filter cartridge 300 via inlet tubing 400. The filter cartridge 300 includes a chamber 310 supporting first and second electrodes 320, 330. The first electrode 320 is coupled to the power supply 1000 via a first ionizer 2010 (FIG. 1) that creates a first electric field around the first electrode 320 having a negative charge. The second electrode 330 is coupled to the power supply 1000 via a second ionizer 2020 (FIG. 1) that creates a second electric field around the second electrode 330 having a positive charge. As the contaminated insufflation gas containing, e.g., smoke and particulate matter, flows through the filter cartridge 300, the contaminated insufflation gas first passes through the first electric field where the airborne particulate matter is ionized by the negative charge present in the first electric field, thereby acquiring a generally negative charge. As the ionized airborne particulate matter flows through the chamber 310 in the direction of arrows "S", it passes into the second electric field that has a positive electric charge. The oppositely charged airborne particulate matter is attracted by the positive electric charge and accumulates in the chamber 310 of the filter cartridge 300 in the vicinity of the second electrode 330. In this manner, evacuating the smoke and particulate matter in the insufflation gas in the body cavity may be performed in parallel with a surgical procedure, following a surgical procedure, or a combination thereof.

By subjecting the airborne particulate matter to a negative electric field and ionizing the airborne particulate matter with a negative electric charge, the oppositely charged electrode easily attracts and retains the ionized airborne particulate matter thereby inhibiting the airborne particulate matter from exiting the body cavity and into the environment surrounding the patient (e.g., an operating room).

It is contemplated that the electrical fields may be reversed such that a positive electric field is generated in the vicinity of the first electrode 320 and a negative electric field is generated in the vicinity of the second electrode 330. In this instance, the airborne particulate matter would acquire a positive electric charge as it passes through the first electrode 320 and is attracted to the negative electric field near the second electrode 330 where the airborne particulate matter would accumulate. One or both of the first and second electrodes 320, 330 may be a wire, a mesh (FIG. 3), a flexible conductive circuit, an electrically conductive organic compound, or combinations thereof. In an aspect, the mesh may be formed of stainless steel.

FIG. 4 illustrates the components of the power module 200. The power module 200 includes the pump 700, first and second ionizer units 2010, 2020 (only one shown), and the power supply 1000. As discussed hereinabove, the pump 700 includes the inlet and outlet ports 720, 740 that are in communication with the filter cartridge 300 and the insufflation source connector 452, respectively, via the respective connecting inlet tubing 480 and connecting outlet tubing 490. The pump 700 is electrically coupled to the power supply 1000 for supply of power. In an aspect, the pump 700 may be a diaphragm pump with pump capacity of about 15 liters per minute (LPM). The first and second ionizer units 2010, 2020 are coupled to the first and second electrodes 320, 330 to create respective electric fields therearound, as discussed hereinabove. The first and second ionizer units 2010, 2020 are electrically coupled to the power supply 1000 for supply of power. In an aspect, the power supply 1000 is a high voltage DC power supply that receives an input voltage of about 12 VDC and has an output voltage between about 10k VDC to 30k VDC. In an aspect, the power supply 1000 is a lithium-ion battery.

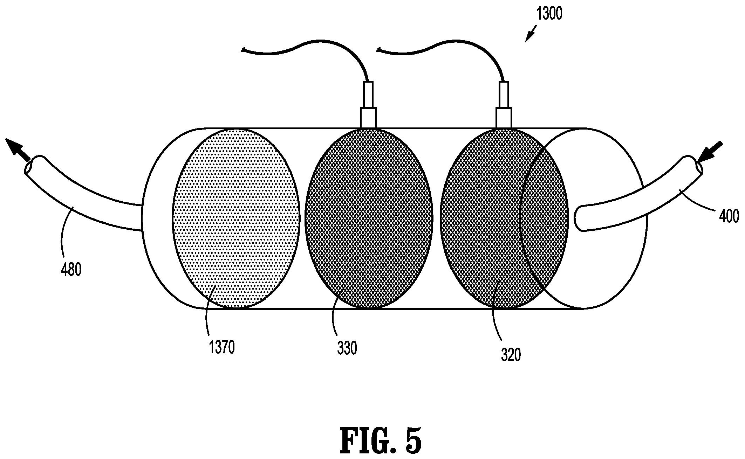

FIG. 5 illustrates a filter cartridge 1300 in accordance with another aspect of the disclosure. The filter cartridge 1300 may be disposable. Similar to the filter cartridge 300, the filter cartridge 1300 includes first and second electrodes 320, 330 that are configured in a manner described hereinabove. However, the filter cartridge 1300 may further include a mechanical filter 1370. The mechanical filter 1370 captures any particulate matter that is not captured by the second electrode 330. In an aspect, the mechanical filter 1370 may be a high efficiency particulate air (HEPA) filter or an ultra-low particulate air (ULPA) filter.

In use, the first and second trocars 500, 550 are placed with respect to the surgical site to maintain proper pneumoperitoneum of the surgical site. Initially, an insufflation gas is supplied to the surgical site by the insufflation source "IS" to inflate the body cavity. The clinician may perform surgical procedures as needed. During the surgical procedure, the clinician may utilize electrosurgical devices that may create smoke and impair visualization of the surgical site. The clinician may activate the smoke filtration system 100 as needed to remove the contaminated insufflation gas safely and efficiently from the body cavity. Activation of the smoke filtration system 100 activates the pump 700 to discharge the contaminated insufflation gas from the body cavity through the second trocar 550 to the filter cartridge 300. The first and second electrodes 320, 330 and optionally the mechanical filter 1370 removes the particulate matters and pathogens from the contaminated insufflation gas. The filtered insufflation gas is returned to the body cavity along with a new insufflation gas from the insufflation source "IS." In this manner, the smoke filtration system 100 inhibits the contaminated insufflation gas from being released to the operating room and the environment, while maintaining pneumoperitoneum and improving visibility of the surgical site.

Persons skilled in the art will understand that the devices and methods specifically described herein and illustrated in the accompanying drawings are non-limiting. The scope of the present invention is defined by the claims that follow.

## Claims

1. A closed loop filtration system (100) for use in a laparoscopic surgical procedure comprising:
first and second trocars (500, 550) providing sealed access to a body cavity;
a power supply (1000);
first and second ionizer units (2010, 2020) electrically coupled to the power supply; and
a filter cartridge (300) including:
an outlet in communication with the first trocar (500);
an inlet in communication with the second trocar (550);
a first electrode (320) disposed downstream of the inlet of the filter cartridge, the first electrode electrically coupled to the first ionizer unit (2010) to ionize airborne particulate matter flowing therethrough; and
a second electrode (330) disposed downstream of the first electrode, the second electrode electrically coupled to the second ionizer unit (2020) and configured to attract the airborne particulate matter that is ionized by the first electrode.

2. The closed loop filtration system according to claim 1, further including a pump (700) connected to the outlet of the filter cartridge to direct a fluid from the second trocar (550) to the filter cartridge.

3. The closed loop filtration system according to claim 2 wherein the pump (700) is electrically coupled to the power supply (1000) and has a pump inlet (720) in communication with the outlet of the filter cartridge and a pump outlet (740) in communication with the first trocar (500).

4. The closed loop filtration system according to claim 2 or 3, further including an insufflation source connector (452) that is coupled to an insufflation source and the first trocar (500).

5. The closed loop filtration system according to claim 4, wherein the insufflation source connector (452) is also connected to an outlet of the pump.

6. The closed loop filtration system as claimed in any preceding claim wherein at least one of the first or second electrodes is formed of stainless steel.

7. The closed loop filtration system according to any preceding claim, wherein the first or second electrode is a mesh.

8. The closed loop filtration system according to any preceding claim, wherein the filter cartridge further includes a mechanical filter (1370).

9. The closed loop filtration system according to claim 8 wherein the mechanical filter (1370) is disposed downstream of the second electrode.

10. The closed loop filtration system as claimed in claim 8 or 9 wherein the mechanical filter (1370) is a HEPA or ULPA filter.

11. The closed loop filtration system according to any preceding claim, wherein the power supply (1000) is configured to provide an output voltage between about 10k VDC to 30k VDC.

12. The closed loop filtration system according to any preceding claim, wherein the power supply (1000) is a lithium-ion battery.

13. The closed loop filtration system according to any preceding claim, wherein the first electrode (320) is negatively charged.

14. The closed loop filtration system according to claim 3, further including an insufflation source connector (452) disposed downstream of the pump outlet (740), the insufflation source connector coupled to an insufflation gas source.

## Patentansprüche

1. Filtrationssystem (100) mit geschlossenem Regelkreis zur Verwendung in einer laparoskopischen chirurgischen Prozedur, das Folgendes umfasst:
einen ersten und ein zweiten Trokar (500, 550), die einen abgedichteten Zugang zu einem Körperhohlraum bereitstellen;
eine Stromversorgung (1000);
eine erste und eine zweite Ionisatoreinheit (2010, 2020), die an die Stromversorgung elektrisch gekoppelt sind; und
eine Filterkartusche (300), die Folgendes enthält:
einen Auslass in Kommunikation mit dem ersten Trokar (500);
einen Einlass in Kommunikation mit dem zweiten Trokar (550);
eine erste Elektrode (320), die stromabwärts des Einlasses der Filterkartusche angeordnet ist, wobei die erste Elektrode an die erste Ionisatoreinheit (2010) elektrisch gekoppelt ist, um luftgetragene Schwebstoffe, die durch sie strömen, zu ionisieren; und
eine zweite Elektrode (330), die stromabwärts der ersten Elektrode angeordnet ist, wobei die zweite Elektrode an die zweite Ionisatoreinheit (2020) elektrisch gekoppelt ist und konfiguriert ist, die luftgetragenen Schwebstoffe, die durch die erste Elektrode ionisiert sind, anzuziehen.

2. Filtrationssystem mit geschlossenem Regelkreis nach Anspruch 1, das ferner eine Pumpe (700) enthält, die mit dem Auslass der Filterkartusche verbunden ist, um ein Fluid aus dem zweiten Trokar (550) zur Filterkartusche zu leiten.

3. Filtrationssystem mit geschlossenem Regelkreis nach Anspruch 2, wobei die Pumpe (700) an die Stromversorgung (1000) elektrisch gekoppelt ist und einen Pumpeneinlass (720) in Kommunikation mit dem Auslass der Filterkartusche und einen Pumpenauslass (740) in Kommunikation mit dem ersten Trokar (500) aufweist.

4. Filtrationssystem mit geschlossenem Regelkreis nach Anspruch 2 oder 3, das ferner einen Einblasquellenverbinder (452) enthält, der an eine Einblasquelle und den ersten Trokar (500) gekoppelt ist.

5. Filtrationssystem mit geschlossenem Regelkreis nach Anspruch 4, wobei der Einblasquellenverbinder (452) auch mit einem Auslass der Pumpe verbunden ist.

6. Filtrationssystem mit geschlossenem Regelkreis nach einem vorhergehenden Anspruch, wobei mindestens eine der ersten oder der zweiten Elektrode aus Edelstahl gebildet ist.

7. Filtrationssystem mit geschlossenem Regelkreis nach einem vorhergehenden Anspruch, wobei die erste oder die zweite Elektrode ein Gitter ist.

8. Filtrationssystem mit geschlossenem Regelkreis nach einem vorhergehenden Anspruch, wobei die Filterkartusche ferner einen mechanischen Filter (1370) enthält.

9. Filtrationssystem mit geschlossenem Regelkreis nach Anspruch 8, wobei der mechanische Filter (1370) stromabwärts der zweiten Elektrode angeordnet ist.

10. Filtrationssystem mit geschlossenem Regelkreis nach Anspruch 8 oder 9, wobei der mechanische Filter (1370) ein HEPA- oder ein ULPA-Filter ist.

11. Filtrationssystem mit geschlossenem Regelkreis nach einem vorhergehenden Anspruch, wobei die Stromversorgung (1000) konfiguriert ist, eine Ausgangsspannung etwa im Bereich von 10 kVDC bis 30 kVDC bereitzustellen.

12. Filtrationssystem mit geschlossenem Regelkreis nach einem vorhergehenden Anspruch, wobei die Stromversorgung (1000) eine Lithium-Ionen-Batterie ist.

13. Filtrationssystem mit geschlossenem Regelkreis nach einem vorhergehenden Anspruch, wobei die erste Elektrode (320) negativ geladen ist.

14. Filtrationssystem mit geschlossenem Regelkreis nach Anspruch 3, das ferner eine Einblasquellenverbinder (452) enthält, der stromabwärts des Pumpenauslasses (740) angeordnet ist, wobei der Einblasquellenverbinder an eine Einblasgasquelle gekoppelt ist.

## Revendications

1. Système de filtration en boucle fermée (100) destiné à être utilisé dans une procédure chirurgicale laparoscopique, comprenant :
des premier et second trocarts (500, 550) fournissant un accès hermétique à une cavité corporelle ;
une alimentation électrique (1000) ;
des première et seconde unités d'ionisation (2010, 2020) couplées électriquement à l'alimentation électrique ; et
une cartouche de filtre (300) comprenant :
une sortie en communication avec le premier trocart (500) ;
une entrée en communication avec le second trocart (550) ;
une première électrode (320) disposée en aval de l'entrée de la cartouche de filtre, la première électrode étant couplée électriquement à la première unité d'ionisation (2010) pour ioniser des matières particulaires en suspension dans l'air circulant à travers ; et
une seconde électrode (330) disposée en aval de la première électrode, la seconde électrode étant couplée électriquement à la seconde unité d'ionisation (2020) et conçue pour attirer les matières particulaires en suspension dans l'air qui sont ionisées par la première électrode.

2. Système de filtration en boucle fermée selon la revendication 1, comprenant en outre une pompe (700) raccordée à la sortie de la cartouche de filtre pour diriger un fluide du second trocart (550) vers la cartouche de filtre.

3. Système de filtration en boucle fermée selon la revendication 2, dans lequel la pompe (700) est couplée électriquement à l'alimentation électrique (1000) et comporte une entrée de pompe (720) en communication avec la sortie de la cartouche de filtre et une sortie de pompe (740) en communication avec le premier trocart (500).

4. Système de filtration en boucle fermée selon la revendication 2 ou 3, comprenant en outre un raccord de source d'insufflation (452) qui est couplé à une source d'insufflation et au premier trocart (500).

5. Système de filtration en boucle fermée selon la revendication 4, dans lequel le raccord de source d'insufflation (452) est également raccordé à une sortie de la pompe.

6. Système de filtration en boucle fermée selon l'une quelconque des revendications précédentes, dans lequel au moins l'une de la première ou de la seconde électrode est formée en acier inoxydable.

7. Système de filtration en boucle fermée selon l'une quelconque des revendications précédentes, dans lequel la première ou la seconde électrode est une électrode à mailles.

8. Système de filtration en boucle fermée selon l'une quelconque des revendications précédentes, dans lequel la cartouche de filtre comprend en outre un filtre mécanique (1370).

9. Système de filtration en boucle fermée selon la revendication 8, dans lequel le filtre mécanique (1370) est disposé en aval de la seconde électrode.

10. Système de filtration en boucle fermée selon la revendication 8 ou 9, dans lequel le filtre mécanique (1370) est un filtre HEPA ou un filtre ULPA.

11. Système de filtration en boucle fermée selon l'une quelconque des revendications précédentes, dans lequel l'alimentation électrique (1000) est configurée pour produire une tension de sortie comprise entre environ 10k V en courant continu et 30k V en courant continu.

12. Système de filtration en boucle fermée selon l'une quelconque des revendications précédentes, dans lequel l'alimentation électrique (1000) est une batterie au lithium-ion.

13. Système de filtration en boucle fermée selon l'une quelconque des revendications précédentes, dans lequel la première électrode (320) est chargée négativement.

14. Système de filtration en boucle fermée selon la revendication 3, comprenant en outre un raccord de source d'insufflation (452) disposé en aval de la sortie de pompe (740), le raccord de source d'insufflation étant couplé à une source de gaz d'insufflation.
